Europäisches Patentamt

European Patent Office

Office européen des brevets

(19)

(11) Publication number: **0 079 777**

A2

(12) **EUROPEAN PATENT APPLICATION**

(21) Application number: **82306040.5**

(22) Date of filing: **12.11.82**

(51) Int. Cl.³: **C 12 P 7/08**
**C 12 M 1/00, C 12 F 1/02**

(30) Priority: **16.11.81 US 321190**

(43) Date of publication of application:
**25.05.83 Bulletin 83/21**

(84) Designated Contracting States:
**AT BE CH DE FR GB IT LI NL SE**

(71) Applicant: **FERMENTEC CORPORATION**
**301 Saratoga Avenue**
**Los Gatos California 95030(US)**

(72) Inventor: **Hybarger, Ray Mott**
**335 Henderson Drive**
**San Jose California 95123(US)**

(74) Representative: **Armitage, Ian Michael et al,**
**MEWBURN ELLIS & CO. 2/3 Cursitor Street**
**London EC4A 1BQ(GB)**

(54) **Waste treatment apparatus and process.**

(57) Aqueous sugar containing wastes are fermented with two batch fermentation tanks (12,14) supplied from a hot storage tank (10). As fermentation proceeds in one tank, the previously fermented contents in the other tank are metered to a distillation column (16) where concentrated, industrial grade ethanol is recovered.

FIG. 1

EP 0 079 777 A2

-1-

## WASTE TREATMENT APPARATUS AND PROCESS

In the past when petroleum was less expensive, industrial grade ethanol was produced from ethylene. Fermentation technology was directed to systems operating on a large industrial scale where preservation of enhancing taste components of alcoholic beverages was a principal objective.

Systems for treating aqueous wastes containing sugar have relied upon traditional sewage treating apparatus combining large aerobic and anaerobic treatment tanks, aerated setting ponds, trickle filters and the like to reduce BOD levels. Recovery of useful by-products has not been a major interest because the systems available were not cost effective, i.e., the cost of installation and operation of any system when compared with traditional alternatives, exceeded the value of the by-products.

This invention is directed to an economical waste treatment apparatus particularly suited to treat sugar containing aqueous wastes of the type produced by soft drink manufacturers and bottlers on site to reduce BOD levels of the waste while producing ethanol as an economical by-product. The apparatus comprises two fermentation vessels and a pump and heat exchanger system. Conduits are provided for recirculating and cooling fermenting liquid in one vessel while the fermented contents of the other vessel are metered to a distillation column and versa in alternating sequence. The liquid waste is fed to the fermentation vessels from a hot storage tank where it has been

-2-

pasteurized by heating at a pasteurizing temperature. The distillation column includes an improved reflux system controlling ethanol concentration of the distillate while assuring that reflux is continued in the event feed of fermented liquid to the column is interrupted, thus protecting the packing and heating system.

According to one aspect of the present invention there is provided a process for treating sugar-containing aqueous waste by fermentation, wherein the aqueous waste is supplied alternately to separate fermentation tanks, so that while fermentation is taking place in one fermentation tank the previously fermented contents of the other fermentation tank are fed to a distillation column. Preferably the aqueous waste is supplied to the fermentation tanks from a hot storage tank.

In another aspect the invention provides waste treatment apparatus suitable for use in the above process.

The process and apparatus will be further illustrated by the following description of specific embodiments with reference to the accompanying drawings, wherein:

Figure 1 is a schematic representation of a simplified system according to this invention.

Figure 2 is a schematic representation of an embodiment of the hot holding tank and associated recycle and heating system;

Figure 3 is a schematic representation of an embodiment of the fermentation system of this invention and associated recirculation and cooling system;

-3-

Figure 4 is a schematic representation of an embodiment of the distillation system of this invention;

Figure 5 is a schematic representation of the improved distillation system of this invention; and

Figure 6 is a detailed schematic representation of the improved reflux drum of this invention.

The present invention is a system for treating waste streams containing sugar such as those typically found in soft drink bottling plants. Such waste streams contain sugar as their major BOD component. To meet the stringent BOD limits legally required by agencies concerned with pollution, it is necessary to reduce the BOD levels. The present system is particularly designed to satisfy this need while creating industrial grade ethanol of high concentration as a useful by-product.

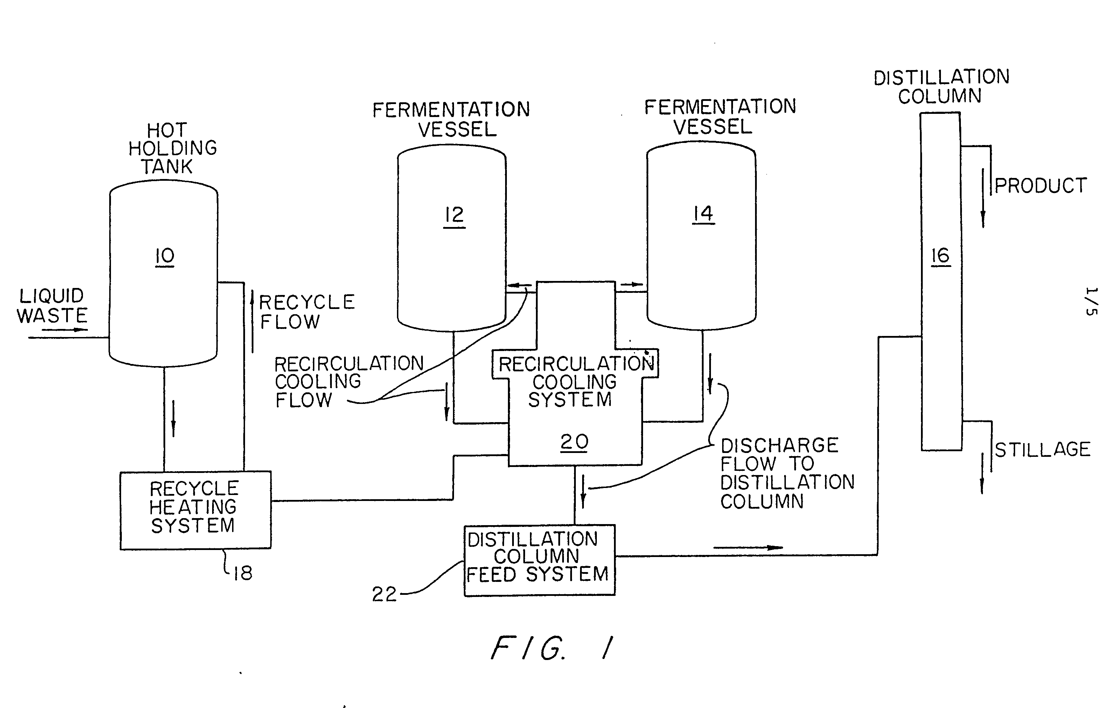

Referring to Figure 1, a diagrammatic representation of an overall system of this invention is shown. The system comprises a hot holding tank 10, fermentation vessels 12 and 14 and distillation column 16. Associated with the hot holding tank 10 is a recycle-heating system 18. Aqueous waste liquid containing sugar is fed into the hot holding tank 10. The aqueous waste liquid containing sugar is heated to a pasteurizing temperature as it is recycled. Storage in the hot holding tank at the elevated temperature pasteurizes the liquid, reducing bacterial growth during storage. The hot holding tank 10 provides a collection point to store high sugar wastes until sufficient liquid is available for a batch. Fermentation tanks 12 and 14 have associated therewith a recirculation-cooling system 20 and

-4-

a distillation column feed system 22. Liquid sugar containing waste from the hot holding tank 10 is transferred to one of the fermentation vessels 12 or 14, mixed with yeast and nutrients and recirculated and cooled until fermentation is substantially complete. The contents of the other fermentation vessel are substantially fermented wastes from the previous operating mode and are metered to the distillation column for separating a highly concentrated industrial grade ethanol therefrom. Meanwhile, the aforesaid fermentation proceeds in the first mentioned fermentation vessel.

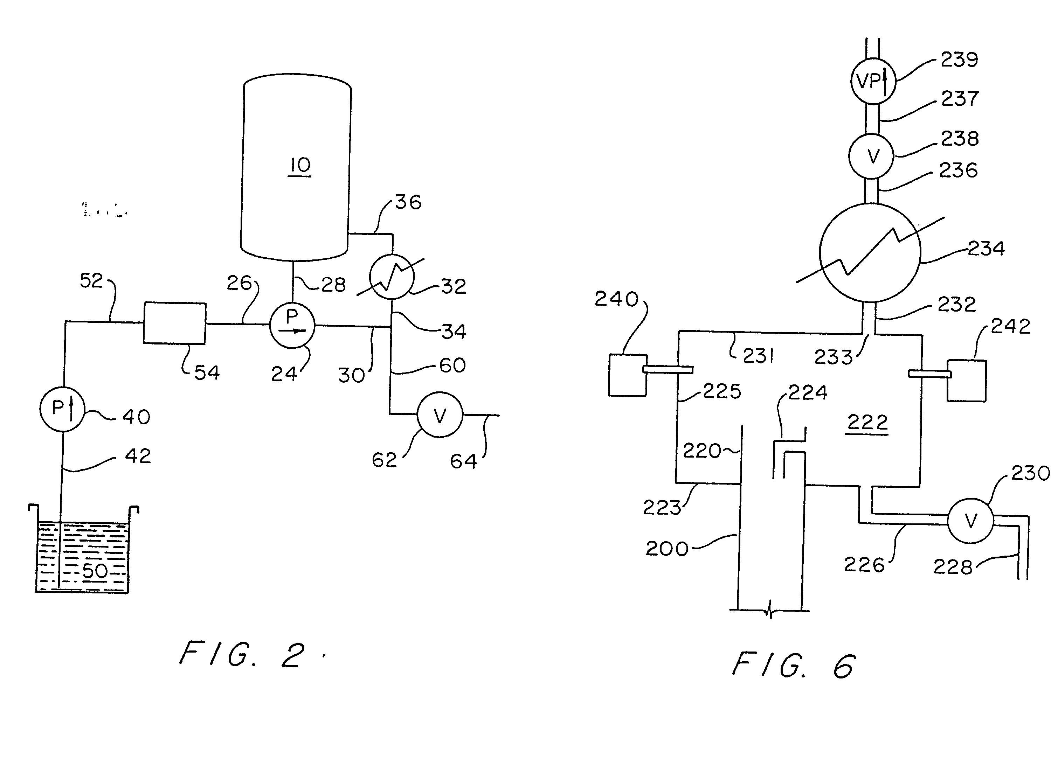

Referring to Figure 2, a schematic representation of an embodiment of the hot holding tank and associated recycle-heating system according to this invention is shown.

The hot holding tank 10 provides a collection point to hold and accumulate high sugar wastes until sufficient liquid is available for a batch. It should be sufficiently large to provide surge capacity for periods when waste flow exceeds the fermentation capacity of the system. The hot holding tank recycle and heating system comprises a pump 24 having inlet conduits 26 and 28 and an outlet conduit 30 communicating therewith. Also included is heat exchanger 32 having inlet conduit 34 and outlet conduit 36 communicating therewith. Alternatively, heat exchanger 32 can be replaced by a direct steam injector for injecting steam into the recycling liquid. The pump inlet conduit 28 is the tank outlet conduit. The heat exchanger outlet conduit 36 connects the heat exchanger 32 with the hot holding tank 10.

Collection sump 50 is connected with sump pump 40 having an inlet conduit 42 and a sump pump outlet conduit 52 communicating with strainer 54. The strainer 54 can be a duplex strainer of a standard type having a mechanism for interposing a clean strainer and removing a fouled strainer without interrupting liquid transfer. The pump inlet conduit 26 is the strainer outlet conduit.

The hot holding tank system is connected with the fermentation tanks by way of conduit 60, transfer valve 62 and conduit 64. For transferring sugar containing liquid waste to the hot holding tank 10, the waste is pumped from collection sump 50 through sump pump inlet conduit 42 by sump pump 40. From there it is fed by sump pump outlet conduit 52 to the strainer 54 and from there to pump 24 through the pump inlet conduit 26. The waste is pumped through the pump outlet conduit 30, heat exchanger inlet conduit 34, heat exchanger 32, and heat exchanger outlet conduit 36 to the hot holding tank 10. The strainer 54 removes gross particulate matter.

For heating and agitating the contents of the hot holding tank 10, liquid waste is pumped from tank 10 through outlet conduit 28 to the pump 24. The liquid waste is directed through pump outlet conduit 30, heat exchanger inlet conduit 34 to the heat exchanger 32 and therefrom through heat exchanger outlet conduit 36 to the tank 10. Sufficient heat is supplied by heat exchanger 32 to maintain the waste liquid temperature in the tank above 140°F. This pasteurizing temperature is sufficient to reduce bacterial level substantially and prevent conversion of sugar during storage to other BOD contributing chemical components which would survive fermentation. Prolonged heating during storage requires less heat energy than rapid sterilization or pasteurization procedures.

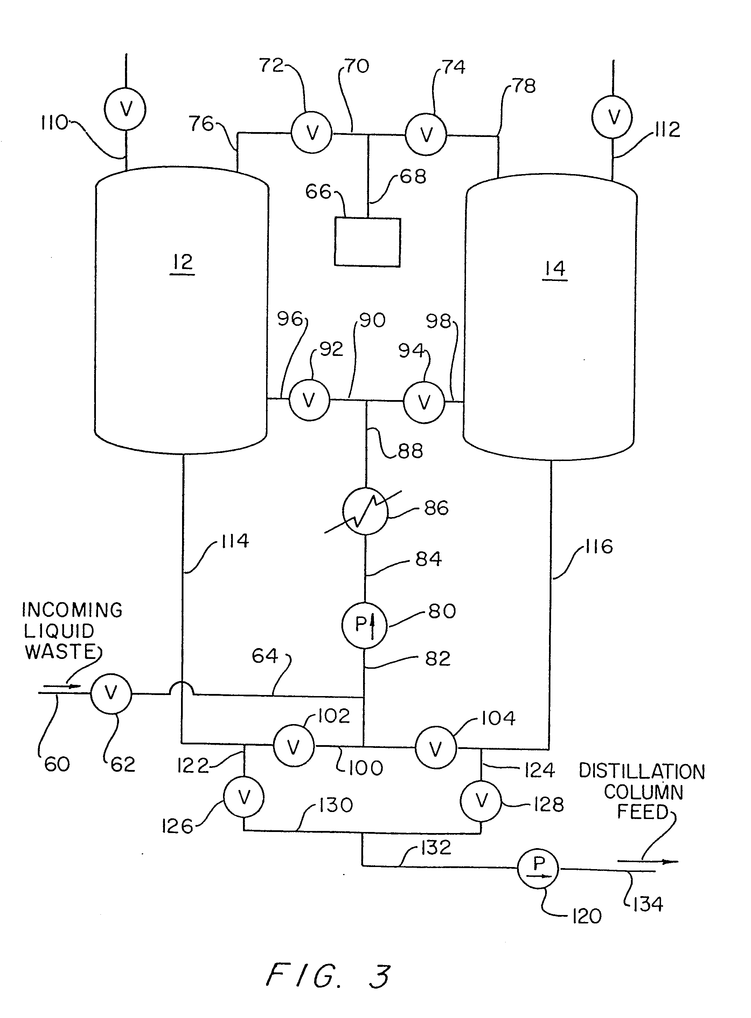

Referring to Figure 3 a schematic representation of an embodiment of the fermentation system of this invention and associated recirculation and cooling system and distillation column feed system is shown. The two fermentation vessels 12 and 14 are batch fermentation tanks, each of which functions alternately as a fermenting vessel and as a reservoir for supplying fermented liquid waste to the distillation column 16. Each fermentation vessel should have the capacity for the average daily waste flow from the facility. As a critical feature of this invention, a single recirculation system effects cooling of recirculating fermenting waste for each fermentation tank during its fermenting mode. This recirculation system comprises a recirculation pump 80 having an inlet conduit 82 and an outlet conduit 84 connected with the heat exchanger 86. The heat exchanger outlet conduit 88 communicates through conduit 90 with inlet control valves 92 and 94 and the respective inlet conduits 96 and 98 connected therewith. Preferably the inlet conduits 96 and 98 terminate in standard spray mixing nozzles (not shown) in the respective fermenter. The recirculating system heat exchanger 86 and pump 80 can be arranged in reverse order to that shown in Figure 3 if desired. In the configuration represented in Figure 3, the recirculating pump inlet conduit 82 is connected by conduit 100 and through recycle control valves 102 and 104 with respective fermentation vessel outlet conduits 114 and 116.

In the embodiment of Figure 3, heated waste is supplied to the fermentation vessels 12 and 14 through the recirculation system. Incoming liquid waste from the hot holding tank 10 is supplied to conduit 60, transfer control valve 62 and through supply conduit 64 and to pump inlet conduit 82. Alternatively, the supply conduit 64 could communicate with outlet conduits 114 and 116. Feed of fermented waste to the metering pump 120 feeding distillation column 16 is effected by a discharge control valve system communicating with outlet conduits 114 and 116 and comprising the recycle control valves 102 and 104, the discharge conduits 122 and 124 connecting the respective outlet conduits 114 and 116 and with the respective discharge control valves 126 and 128. The discharge control valves 126 and 128 communicate through conduit 130 with the metering pump inlet conduit 132, the metering pump 120 and thereby with the distillation column feed conduit 134.

Anti-foam agents can be supplied from tank 66 by anti-foam agent supply conduits 68 and 70. Conduit 70 is connected through control valves 72 and 74 and through respective inlet conduits 76 and 78 to the respective fermentation vessels 12 and 14. Anti-foam supply tank 66 can be replaced with separate tanks, each connected with only one fermentation vessel. Nutrients and yeast can be added to the fermentation vessels 12 and 14 through respective feed means 110 and 112 which can be conduits for liquids or handholes for dry solids, for example.

Heated waste liquid containing fermentable sugar is supplied to an empty fermentation vessel by the following procedure. Referring to Figure 2, heated waste liquid from the hot holding tank 10 is directed through outlet conduit 28, pump 24, pump outlet conduit 30, conduit 60 and through transfer control valve 62 to conduit 64.

Referring to Figure 3 the following is a description of a first operating mode which is initiated when fermentation vessel 14 contains fermented waste ready for distillation and fermentation vessel 12 is empty, its contents having been fed to the distillation column 16 in the immediately previous operating mode. Recycle control valve 104, inlet control valve 94 and discharge

-8-

control valve 126 are closed. Heated liquid waste at a temperature above 140°F is directed from the open transfer control valve 62 through conduit 64 to the pump inlet conduit 82, pump 80, pump outlet conduit 84, through preferably inactive heat exchanger 86, heat exchanger outlet conduit 88, conduit 90, open inlet control valve 92 and inlet conduit 96 to fermentation tank 12. Flow continues until the fermentation vessel 12 is filled. During the filling operation, liquid is preferably recirculated from tank 12 through outlet conduit 114 to pump inlet conduit 82. The associated recirculation system and fermentation tank 12 are thus exposed to the hot waste from the hot holding tank before it is cooled, thus reducing the bacterial contamination in the system substantially. This reduces the frequency of decontamination with steam or chemicals. The latter increases costs and reduces operating time of the system since special decontamination procedures would require taking the system out of operation.

To effect the recirculation and cooling cycle, transfer control valve 62 is closed. Liquid flow is continued from fermentation vessel 12 through outlet conduit 114, recycle control valve 102, pump inlet conduit 82, pump 80, pump outlet conduit 84, activated heat exchanger 86, heat exchanger outlet conduit 88, conduit 90, inlet control valve 92, and through inlet conduit 96 to fermentation vessel 12. Undesirable heat is removed by liquid passage through heat exchanger 86. When the liquid temperature is below 95°F, yeast and any desired nutrients or other chemicals are added through feed means 110. The additives used are generally known in fermentation. If dry yeast is used, the amount added is preferably within the range of from 0.5 to 2.0 grams per gallon of waste liquid. Typical yeast nutrients are water soluble inorganic or organic chemicals providing nutrient forms of nitrogen such as ammonium ions, urea, potassium, phosphates and the like. Diammonium phosphate, potassium nitrate or other chemicals may be added. pH adjustment can be made by adding an inorganic base such as ammonium hydroxide or an inorganic acid such as hydrochloric acid or phosphoric acid. The preferred pH range is from 4 to 5. As the fermentation proceeds, sufficient heat is removed from liquid circulating through heat exchanger 86 to maintain the fermenting liquid temperature within the range of from 85°F to 95°F.

Meanwhile during the first operating mode, fermented waste liquid is fed from fermentation vessel 14 to the distillation column 16 by metering pump 120. The discharge control valve 128 is positioned in the open position, and fermented waste liquid is fed through outlet conduit 116, conduit 124, discharge control valve 128, conduits 130, metering pump inlet conduit 132, metering pump 120 and through conduit 134 until fermentation vessel 14 has been emptied.

The first operating mode is ended when fermentation in fermentation vessel 12 is complete and fermentation vessel 14 has been emptied. The second operating mode is then initiated and is identical to the first operating mode except the fermentation vessel 12, being now filled with fermented waste, functions to supply the distillation column 16 while fermentation vessel 14 functions as a fermenter. This operation will be briefly described, it being understood that the details provided regarding the first operating mode are equally applicable to the second operating mode.

By placing recycle control valve 102, discharge control

-10-

valve 128, inlet control valve 92 in a closed position and transfer control valve 62, recycle control valve 104, and inlet control valve 94 in an open position, flow of hot waste liquid is directed in sequence through conduit 60, transfer valve 62, conduit 64, pump inlet conduit 82, recirculating pump 80, outlet conduit 84, inactive heat exchanger 86, heat exchanger outlet conduit 88, conduit 90, inlet control valve 94 and inlet conduit 98 to the fermentation vessel 14. Circulation of hot waste liquid from vessel 14 through conduit 116 and valve 104 to inlet conduit 82 is preferred.

When the fermentation vessel 14 is filled, the recirculation and cooling cycle is effected. Transfer valve 62 is closed and the heat exchanger 86 is activated to effect cooling. Anti-foam chamicals are supplied through conduit 78, and nutrients and pH control chemicals are added through inlet means 112. The above-described fermentation cycle is thus initiated. Supply of fermented waste from fermentation vessel 12 to the metering pump 120 is established by placing discharge control valve 126 in an open position, inlet control valve 92, recycle control valve 102 and discharge control valve 128 remaining in a closed position.

The shared recirculation-cooling system allows low capital costs, not only because only one pump and one heat exchanger are used but because any control system needed can be associated with the common system. For example, controllers associated with the liquid condition such as instruments detecting temperature, pH, alcohol concentration, yeast concentration and the like can have their detectors in the common part of the circuit. Duplication is thus avoided.

-11-

In the optimum operating cycle, the apparatus of this invention is operated on a 24-hour cycle, that is, each operating mode is approximately 24-hours in duration. One of the particular advantages of this dual system is the extended fermentation time achieved. Fermentation of waste liquid proceeds from beginning to near completion in a first mode and continues in the second mode until the liquid is discharged. On a 24-hour cycle, an effective fermentation time of almost 36 hours is thus achieved. Since fermentation of residual sugars is continuing at a greatly reduced rate in the fermentation vessel functioning as the distillation column supply, recirculation for cooling is not needed. Alternation of the fermentation vessels between active phase (fermentation) and passive phase (distillation column feed or beer feed) allows periodic cleaning through introduction and circulation of hot waste liquid from the hot hold tank. This prevents build up of microorganisms in a beer feed tank, a common occurrence in conventional systems. Also, if special chemical or heat (steam) contamination is required, the distillation operation is not necessarily interrupted since the fermentation vessels can be decontaminated in sequence.

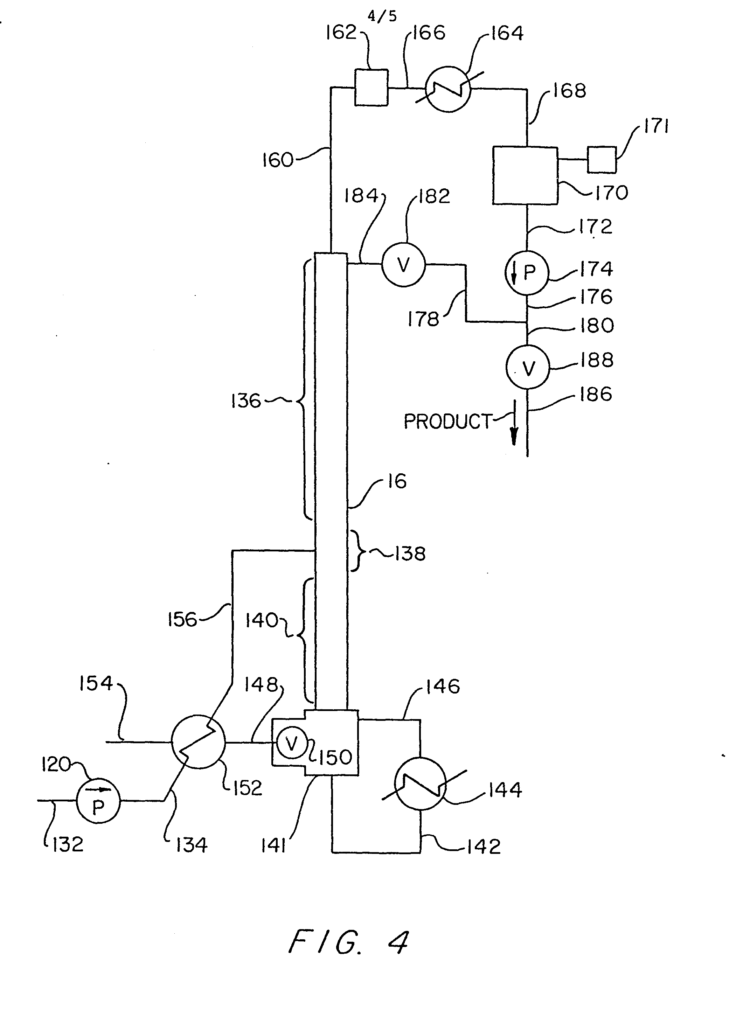

Referring to Figure 4, a schematic representation of an embodiment of the distillation system of this invention is shown. The distillation column 16 is a packed column having an upper packing zone 136 constituting an enriching section where the ethanol purity is increased, a feed zone 138 to which fermented waste liquid is fed through column feed conduit 156 connected thereto, and a lower packing zone 140 constituting a stripping section. The packing material is conventional and can be pall rings, beryl saddles or high efficiency metal mesh, for example. The packed column is more economical than a tray column. Particular

-12-

when soft drink bottling company waste is to be treated, trays are not needed since the waste acts as a cleaning solution, i.e., the system is self-cleaning. High efficiency metal mesh packing is preferred, particularly if the column is to be operated under a vacuum so as to produce anhydrous ethanol.

The column base 140 has communicating therewith a reboiler system comprising reboiler inlet conduit 142, reboiler 144 and reboiler outlet conduit 146. Column stillage outlet conduit 148 communicating with the column base 141 through float control valve 150 connects with a first side of preheater 152. The outlet of the first side of the preheater 152 is the preheater stillage outlet conduit 154. Heat exchange inlet conduit 134 connects the metering pump with the second side of the preheater 152. Column feed conduit 156, constituting the outlet conduit of the second side of the preheater 152 connects the preheater 152 with the column feed zone 138.

The top of the distillation column 16 has associated with it the condenser-reflux system which condenses ethanol as product condensate and controls the ethanol purity of the product condensate by adjusting reflux flow. In the embodiment of Figure 4, this system comprises a vapor outlet conduit 160 leading from the top of column 16 to the vapor purity detector 162 and to the condenser 164 through condenser inlet conduit 166. The vapor purity detector 162 can be a standard differential vapor pressure transmitter, temperature transmitter, or a gas chromatograph, for example. The condenser outlet conduit 168 leads to the reflux condensate accumulator 170 which may be of conventional type. The reflux condensate accumulator can include a level controller 171 controlling liquid level in the accumulator 170 by varying outflow from the system through condensate product conduit 180. This outflow can be reduced by throttling product control valve 188 or reflux control valve 182 as is explained in greater detail hereinafter. The reflux condensate accumulator outlet conduit 172 leads to pump 174. Pump outlet conduit 176 connects with reflux return conduit 178 and condensate product conduit 180. The reflux return conduit communicates with the top of the column 16 through reflux control valve 182 and reflux inlet conduit 184. The condensate product conduit 180 leads to product outlet 186 through conduit product control valve 188.

The vapor purity detector, level controller 171 and associated valves 182 and 188 can be either manual or automatically operating valves. In the latter arrangement, conventional pneumatic or electrically matched detectors, level controllers and respective automatically operated valves would be chose, connected by appropriate tubing or electrical wiring (not shown).

The distillation system functions to remove ethanol from the fermented waste liquid and purify it to a concentrated, industrial grade greater than 180 proof. The fermented waste is pumped by the variable speed metering pump 120 through conduit 134 to the second side of a heat exchanger 152 where it is preheated by heat obtained from the stillage. The stillage is directed through the first side of the heat exchanger 152 by stillage out conduit 148. Preheated fermented waste liquid is then fed by conduit 156 into the feed zone 138, being intimately contacted with rising vapors as it descends through the packing in the stripping section 140 to the column base 141. Liquid in the column base 141 is heated by passage through the reboiler 144.

Vapor rising through the packing of the enriching section

-14-

136 intimately contacts the descending condensed reflux liquid, leading to increase in ethanol concentration in the vapor as it proceeds to the top of the column 16. The vapor passes through conduit 160 and communicates with vapor purity detector 162 which measures a physical property of the vapor indicative of the ethanol concentration and controls the ethanol concentration of the product as will be explained in greater detail hereinafter.

The vapor is condensed to liquid in condenser 164, and the liquid condensate is collected in reflux condensate accumulator 170. From the accumulator 170, a portion of the condensate is pumped through the reflux return conduit 178, reflux control valve 182 and reflux inlet conduit 184 to the top of the column 16 while the other portion is pumped to a product outlet 186 through control valve 188.

The ethanol concentration in the product is controlled by controlling the reflux flow, that is by controlling the relative ratio of the flow of the product and reflux streams. Conventional control systems are described by F.G. Shinskey in Distillation Control for Productivity and Energy Conservation, 1977, published by McGraw-Hill, which is incorporated herein in its entirety by reference.

In a reflux ratio control system, the reflux control valve 182 is controlled in response to the vapor purity as indicated by the vapor purity detector 162. In response to a decrease in detected ethanol level, reflux control valve 182 is opened to increase the reflux flow. Product flow through conduit 180 is controlled in response to the level indicated by level controller 171 by opening condensate control valve 188 as the liquid level in the reflux accumulator 170 increases.

In a material balance control system, condensate product control valve 188 is controlled in response to vapor purity as indicated by vapor purity detector 162. In response to a signal from the vapor purity detector 162 indicating decreasing ethanol concentration in the vapor, condensate control valve 188 is closed to reduce product flow to outlet 186. In that event, reflux flow through valve 182 to the top of the column 16 is controlled by level controller 171 in response to an increasing or decreasing liquid level in the reflux condensate accumulator 170. The material balance control method protects the product purity level from influence by variations in feed composition, feed rate, heat input, or heat removal rate or loss rate. If automatic operation is desired, conventional matched electrical or pneumatically controlled values and level control and vapor pressure detector controllers can be used.

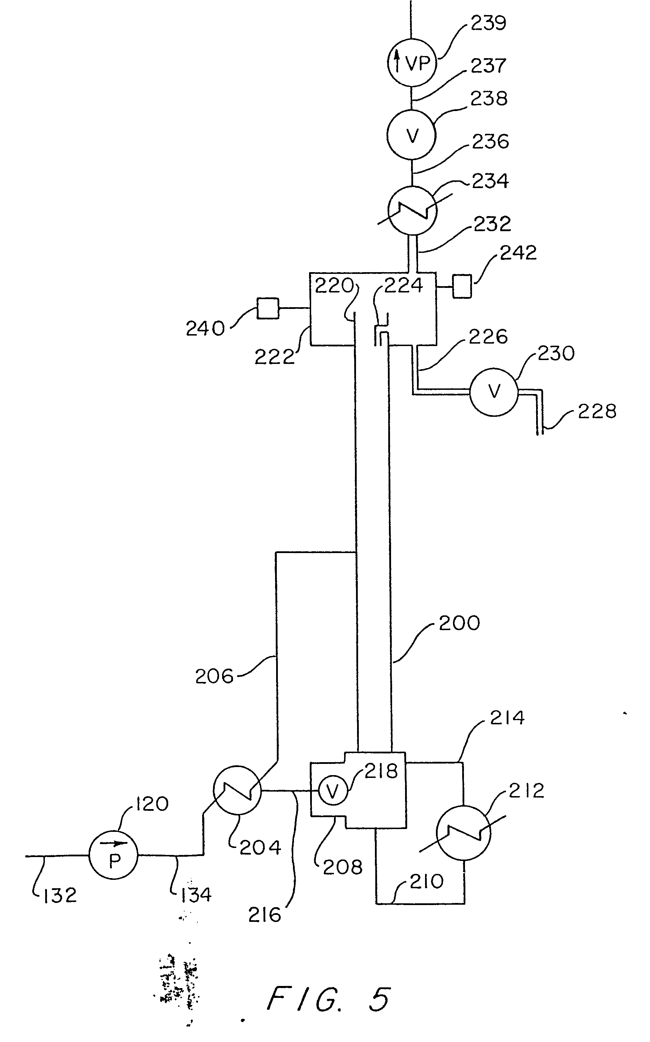

Referring to Figure 5, a schematic representation of an improved distillation column of this invention is shown. The distillation column 200 is a packed column having the components described hereinabove with regard to Figure 4. Fermented liquid waste containing ethanol is metered by pump 120 through heat exchange inlet conduit 134, heat exchanger 204 and column feed conduit 206 to the feed zone of the column 200. Liquid in the column bottom 208 is circulated through reboiler inlet conduit 210 to the reboiler 212 where it is heated before returning to the column bottom through inlet conduit 214. Stillage from the column bottom 208 passes through float control valve 218 and is directed through the preheater 204 by stillage conduit 216. The upper part of the column 200 communicates with the improved reflux drum of this invention.

Reference is directed to Figure 6 for a more detailed representation of the improved reflux drum shown schematically in Figure 5. The reflux drum 222 has a floor 223, an outer wall 225 and a cylindrical partial inner wall 220 communicating with the top of the distillation column 200. Inner wall 220 projects through and above the floor 223 and forms a weir therewith. Overflow conduit 224 in the inner wall 220 is positioned above the floor 223 and constitutes a means for directing condensate flow to the top of the column when the condensate level rises to a level above the overflow conduit 224.

A condenser 234 has an inlet conduit 232 communicating with the reflux drum 222, the condenser being placed at a level above the reflux drum overflow conduit 224. In the condenser, vapor rising from the column 200, through the cylindrical inner wall 220 and through condenser inlet conduit 232 to the condenser 234 is condensed and returned by gravity through conduit 232 to the reflux drum 222.

Product outlet conduit 226 communicates with outlet 228 through condensate product flow control valve 230. The vent conduit 236 and vent control valve 238 communicate with the condenser 234. Vacuum pump 239 is an optional item and may be connected by its inlet conduit 237 with vent control valve 238. It is used when carrying out the distillation at subatmospheric pressures. Pressure sensor 240 communicates with the interior of the reflux drum 222 and indicates the pressure therein. Vent control valve 238 is controlled in response to the pressure indicated so as to maintain steady pressure in the reflux drum 222. Product quality control sensor 242 communicates with the reflux drum 222 and measures the ethanol concentration in the vapor. Its measurements are used to determine the appropriate setting for the condensate product flow control valve 230 so as to control product flow and thereby obtain the desired ethanol concentration in the vapor. The reflux drum 222 has a top wall 231 with a vapor outlet opening 233 communicating with the condensate inlet conduit 232. The vapor outlet opening is positioned vertically over a portion of the reflux drum floor 223 other than over the cylindrical inner wall 220 so that condensate falls by gravity into the reflux drum 222 for return by overflow through overflow line 224 or is removed through product outlet conduit 226.

The pressure sensor 240 and product quality control sensor 242 and associated vent control valve 238 and condensate product flow control valve 230 can be either manual or automatic. In the latter instance, conventional pneumatic or electrically operated sensors and valves can be used, functionally connected by appropriate tubing or electric wiring (not shown).

In the improved reflux drum of this invention, condensate level in the drum 222 is maintained at a constant level by fixed overflow conduit 224, and ethanol concentration is regulated by controlling product control valve 230 in response to ethanol concentration in the vapor as measured by sensor 242. If an ethanol concentration above 190 proof is desired, such as for use in gasohol fuel in low temperature regions, the pressure in the distillation column is reduced by activating vacuum pump 239 and setting vent control valve 238 so as to achieve the desired pressure as indicated by pressure sensor 240.

The improved reflux system of this invention is very effective in on site waste liquid treating systems, particularly those having the size suitable for soft drink bottling plants. It is substantially less expensive than previously known systems performing the same functions. The improved reflux drum of the invention eliminates the need for a level and outside temperature

-18-

control system used with systems having a reflux drum separate from the column, the reflux recirculation pump, and the associated piping and valves.

The operation of the apparatus of the invention is illustrated by the following specific but non-limiting example.

### Example

A bottling company producing a daily average of 1000 gallons of aqueous waste liquid having an average sucrose concentration of 10 percent by weight, pumps the waste liquid from a collecting sump to a hot hold tank having a capacity of 2500 gallons. The incoming waste liquid is introduced into the recycling stream from the hot hold tank. The hot hold tank contents are maintained at a temperature of 160°F by direct steam injection into the recyling stream.

At 24 hour intervals, a charge of 1000 gallons of heated waste liquid is transferred from the hot hold tank to an empty fermenter. As the hot liquid is transferred, the recirculating pump of the fermentation system is activated, thus circulating hot waste through the recirculation system and through an internal mixing nozzle throughout the interior of the fermenter. After 15 minutes of recirculation of the hot liquid, the heat exchanger in the recirculation system is activated and the entering hot liquid is cooled.

Transfer of hot waste is stopped when transfer of a full fermenter charge of 1000 gallons is completed. The liquid is cooled by the heat exchanger to 95°F. At that time, the pH of a sample of the liquid is measured. If the pH is below 4.0, sufficient 10 percent by weight

-19-

ammonium hydroxide solution is added to raise the pH to 4.5. In addition, a charge of two pounds of dry activated yeast powder and one pound of urea is added to the fermenter. Fermentation is allowed to continue under conditions of circulation mixing and temperature control at 90 to 95°F for 22 hours.

At the end of this time, recirulation is stopped, and the liquid contents of the recirculation loop are drained to the distillation column through the distillation column feed pump. The recirculation loop is shut off completely from the distillation feed system. The fermenter contents are directed to the distillation feed loop via a transfer valve and the fermenter is used as a beer feed tank for the distillation column for the next 24 hours.

The distillation column feed pump is adjusted to pump 0.7 gallons per minute of fermented waste to the distillation column. The waste contains approximately 5 weight percent ethanol. The feed stream is heated from 95°F to 215°F in a preheat heat exchanger and fed to the feed section of the distillation column.

Ethanol is stripped from the waste liquid by the section of the column below the feed section. The stripped beer containing less than 0.2 weight percent ethanol and having a temperature of 240°F is withdrawn and fed to the preheat heat exchanger where it is cooled by the incoming feed to below 115°F.

The ethanol above the feed plate is purified to about 190 proof at the top of the column. The ethanol vapor passes a differential vapor pressure sensor which compares

-20-

the pressure of the product vapor with the vapor pressure of a reference standard of ethanol at the same temperature. This differential pressure is used to determine the setting of a central valve in the conduit withdrawing product ethanol from the system. As the purity of the vapor approaches the reference standard, the valve is opened. As the purity decreases, the valve is closed. The top of the column is maintained at a constant pressure of 5 inches of water by an adjustment of a vent control valve in response to a measurement of vapor pressure by a pressure sensor. The ethanol vapor enters a water-cooled heat exchanger above the column where it is condensed, and the liquid ethanol falls into a condensate receiver or reflux drum at the top of the column. The condensate withdrawal as product is controlled by adjusting a valve in response to the differential vapor pressure as described above. The remainder of the condensate is returned to the column through an overflow pipe as reflux.

Thus it will be seen that this invention is capable of providing a compact, total waste liquid fermentation and distillation system which can be constructed and operated at less cost than previously known systems. It is also capable of providing a system for recovering industrial grade ethanol in high concentrations from sugar containing liquid wastes in the process of reducing the BOD and thereof. It is further capable of providing a system for the foregoing purposes suitable for use in a typical soft drink manufacturing or bottling plant with a minimum manpower requirement.

CLAIMS:

1. A waste treatment apparatus comprising:

(a) first and second fermentation vessels (12,14);

(b) a recirculation system (20) comprising a pump (80) and heat exchanger means (86) communicating with an inlet recirculation conduit (82) and an outlet recirculation conduit (88) for recirculating fermenting liquid and removing heat therefrom; and

(c) a conduit system comprising:

(i) a first outlet conduit (114) communicating with said first fermentation vessel (12) and with said inlet recirculation conduit (82);

(ii) a second outlet conduit (116) communicating with said second fermentation vessel (14) and with said inlet recirculation conduit (82);

(iii) a first inlet conduit (96) communicating with said first fermentation vessel (12) and with said outlet recirculation conduit (88);

(iv) a second inlet conduit (98) communicating with said second fermentation vessel (14) and with said outlet recirculation conduit (88); and

(v) valve means (92,94) in said first and second inlet conduits (96,98) for directing liquid recirculation from the first fermentation vessel (12) through the first outlet and inlet conduits (114,96) and back into the first fermentation vessel (12) during a first mode while preventing liquid recirculation from the second fermentation vessel (14) through the second outlet and inlet conduits (116,98), and for directing liquid recirculation from the second fermentation vessel (14)

(116,98)
through the second outlet and inlet conduits/and

(14)
back into the second fermentation vessel/during a

second mode while preventing liquid recirculation

(12)
from the first fermentation vessel/through the

first outlet and inlet conduits (114,96).


2.  The apparatus of Claim 1 comprising:

(a)  pump/and preheater means/having an outlet feed conduit (156)
    (120)        (152)

    (138)
    communicating with the feed inlet/of a distillation

    (16)                    (132)
    column/and an inlet feed conduit/communicating

    (122)
    a third outlet conduit/of said first fermentation

    (12)                  (124)
    vessel/and a fourth outlet conduit/of said second

    (14)
    fermentation vessel/for feeding a controlled volume of solution from a fermentation vessel, preheating it and feeding it to the distillation column; and

    (126,128)                        (122)
(b)  valve means/in said third outlet conduit/and fourth

    (124)
    outlet conduit/for directing liquid flow from the

    (14)
    second fermentation vessel/through the inlet feed

    (132)
    conduit/while preventing liquid flow from the first

    (12)
    fermentation vessel/through the inlet feed conduit during said first mode, and for directing liquid flow

    (12)
    from the first fermentation vessel/through the inlet

    (132)
    feed conduit/while preventing liquid flow from the

    (14)
    second fermentation vessel/through the inlet feed conduit during said second mode.


3.  The apparatus of Claim 2 wherein first and third

(114,122)                            (12)
outlet conduits/of said first fermentation vessel/are the same

(116,124)
conduit and the second and fourth outlet conduits/of said second

(14)
fermentation vessel/are the same conduit.

-23-

4.  The apparatus of Claim 1 comprising:

(a)  a hot waste liquid holding tank (10);

(b)  waste liquid recirculating and heating means (18) /communicating with the tank (10) /for removing liquid thereform, heating it and returning it to the tank; and

(c)  conduit means (60,64) /including valve means (62) /communicating with the waste liquid recirculating and heating means (18) and with said recirculation system (20) /for transferring hot waste liquid from the holding tank (10) /to said recirculation system (20).


5.  The apparatus of Claim 2 comprising:

(a)  a hot waste liquid holding tank (10);

(b)  waste liquid recirculating and heating means (18) /communicating with the tank (10) /for removing liquid therefrom, heating it and returning it to the tank; and

(c)  conduit means (60,64) /including valve means (62) /communicating with the waste liquid recirculating and heating means (18) /and with said recirculation system (20) /for transferring hot waste liquid from the tank (10) /to said recirculation system (20).


6.  The apparatus of/Claim 5 claim 2 or wherein/ (Figs. 5 and 6) the distillation column (200) /has a feed inlet means (206), stillage outlet means (216), reboiler means (212) /and a condenser-reflux means, the condenser reflux means comprising:

(a)  a closed reflux drum (222) /having a floor (223) /and an outer wall (225), cylindrical inner wall means (220) /communicating with the top of the distillation column (200) /and projecting above the reflux drum floor (223) /and forming a weir therewith, an overflow conduit means (224) /in said weir above the reflux drum floor for directing condensate flow to the top of the column when the condensate level rises to a level above the overflow conduit;

(b) a condenser means/ (234) having an inlet conduit/ (232) communicating with the reflux drum/ (222), said condenser being at a level above the reflux drum overflow/ (224) and constituting means for condensing vapor rising from the column and returning condensate to the reflux drum;

(c) a product quality control sensor means/ (242) communicating with the reflux drum at a level above the overflow conduit/ (224) for measuring ethanol concentration in the vapor; and

(d) product outlet conduit means/ (226) including a flow control valve means/ (230) communicating with the reflux drum (222) at a level below the overflow conduit/ (224) for removing condensate product from the reflux drum.

7. The apparatus of Claim 6 wherein reflux drum has a top wall/ (231) with a vapor outlet opening/ (233) communicating with the condenser inlet conduit/ (232), and the vapor outlet opening/ (233) being located vertically over a portion of the reflux drum floor other than over said cylindrical inner wall means (220).

-25-

8. A distillation column (200) having a feed inlet means (206), stillage outlet means (216), reboiler means (212) and a condenser-reflux means, the condenser reflux means comprising:

(a) a closed reflux drum (222) having a floor (223) and an outer wall (225), cylindrical inner wall means (220) communicating with the top of the distillation column (200) and projecting above the reflux drum floor (223) and forming a weir therewith, an overflow conduit means (224) in said weir above the reflux drum floor for directing condensate flow to the top of the column when the condensate level rises to a level above the overflow conduit;

(b) a condenser means (234) having an inlet conduit (232) communicating with the reflux drum (222), said condenser being at a level above the reflux drum overflow (224) and constituting means for condensing vapor rising from the column and returning condensate to the reflux drum;

(c) a product quality control sensor means (242) communicating with the reflux drum at a level above the overflow conduit (224) for measuring ethanol concentration in the vapor; and

(d) product outlet conduit means (226) including a flow control valve means (230) communicating with the reflux drum (222) at a level below the overflow conduit (224) for removing condensate product from the reflux drum.

9. The distillation column of Claim 8 wherein reflux drum has a top wall (231) with a vapor outlet opening (233) communicating with the condenser inlet conduit (232), and the vapor outlet opening (233) being located vertically over a portion of the reflux drum floor other than over said cylindrical inner wall means (220).

10.  A process for treating sugar-containing aqueous waste by fermentation, characterised in that the aqueous waste is supplied alternately to separate fermentation tanks, so that while fermentation is taking place in one fermentation tank the previously fermented contents of the other fermentation tank are fed to a distillation column.

FIG. 1

FIG. 2

FIG. 6

FIG. 3

FIG. 4

FIG. 5